# EUROPEAN PATENT SPECIFICATION

(11) **EP 3 151 775 B1**
(45) Date of publication and mention of the grant of the patent: **01.07.2020**
(21) Application number: 15722865.1
(22) Date of filing: 04.05.2015
(51) Int. Cl.: A61B 46/10, A61B 46/00, A61B 46/23, A61B 46/13

(54) **SURGICAL DRAPE WITH STERILE SYSTEM ACCESS**
CHIRURGISCHES ABDECKTUCH MIT STERILEM SYSTEMZUGRIFF
CHAMP OPÉRATOIRE AVEC ACCÈS DE SYSTÈME STÉRILE

(30) Priority: 27.05.2014 US 201414287563
(43) Date of publication of application: 12.04.2017
(73) Proprietor: O&M Halyard International Unlimited Company, Dublin D15X 98N (IE)
(72) Inventor: LIVESEY, Richard I., Oxfordshire OX33 1PS (GB)
(74) Representative: Dehns
(86) International application number: PCT/US2015/029005
(87) International publication number: WO 2015/183474

(56) References cited:
- WO-A2-2012/078620
- US-A- 4 334 529
- US-A- 5 074 316
- US-A1- 2003 205 233
- US-A1- 2010 319 713
- US-A1- 2012 312 308

## Description

### BACKGROUND OF THE INVENTION

Various configurations of disposable surgical drapes are known in the art for keeping a surgical site on a patient sterile during a surgical procedure. A reinforcement area is often placed around a fenestration or an edge of disposable surgical drapes to provide structural strength and to absorb bodily fluids from the surgical site. Many disposable drapes also include a number of layers of different materials for the drape area and reinforcement area, with each layer providing a different property to the drape. For example, spunbond fabrics, meltblown fabrics, and polymer films have been used as layers in disposable drapes.

Certain surgical procedures involve the injection of contrast fluids. Past practice has been to inject the fluid with a hand held syringe into a line introduced into the femoral or radial artery. Contrast fluid is very viscous so relatively high pressure must be used to infuse the fluid and this requires good hand strength on the part of the physician. Contrast fluid is also expensive and any unused portion of the fluid must be disposed of since it will have entered the patient field during surgery. A relatively new device is becoming more widely used and allows for the saving of the contrast fluid not used on the patient. This device, known as the ACIST CVi contrast delivery system, injects the contrast fluid via a high pressure line from the ACIST system and is controlled by a simple hand control piece, therefore making it less strenuous for the physician to infuse. In addition, any remaining contrast media can be used for another patient as it is stored in a small glass bottle on the ACIST system, outside of the patient field. The ACIST system has a touch screen display (CRT) and lines to deliver the fluid to the patient and a line to allow for the hand control of the fluid flow.

The problem that has arisen is the connection between the system and the patient, since the line(s) between them must pass from inside the patient field to outside the patient field. One practice that has arisen to address this problem is to form holes through the drape with a pen or a sharp instrument and run the controller line and the high pressure line through the holes, with the CRT remaining uncovered and outside the sterile field. Another solution has been to run the line above the drape with no specific barrier between the sterile field and the non-sterile field, hanging it from the operating room ceiling or a piece of equipment. Neither solution is ideal and there is no standard draping system for this system and procedure.

As such, a need currently exists for a surgical drape that has access provided for use with an ACIST CVi or similar system that is easy to use and provides separation between the patient and the equipment.

Related art includes WO2012078620 which discloses a portal for medical instruments. US4334529 discloses a sterile disposable drape. US2003/0205233 discloses a surgical drape and panel assembly. US5074316 discloses a brachial angiography surgical drape. US2010/031713 and US2012/0312308 disclose other surgical drape arrangements.

### SUMMARY

Various features and advantages of the disclosure will be set forth in part in the following description, or may be obvious from the description, or may be learned from practice of the disclosure.

In one aspect of the present invention, there is provided a drape as claimed in claim 1. Preferred embodiments of the invention as claimed in claim 1 are defined by dependent claims 2-12.

In embodiments, the drape includes a material sheet having a size and configuration for covering at least a portion of the patient as well as an instrument array, during the procedure. In an embodiment, the drape includes pre-formed holes allowing the lines from the instrumentation array to the patient to pass through the drape. In an embodiment, the flap covers the holes after the lines have been passed through the holes, in order to help maintain the sterile field around the patient.

In a further embodiment, the instrument array can be a contrast delivery system for angiography.

In an additional embodiment, the drape can be a liquid impermeable material.

In still another embodiment, the drape can include a second hole panel that includes a second hole containing portion located in an alternative location of the drape from the first hole containing portion.

In still another embodiment, the drape can include a base sheet and at least one reinforcement panel, wherein the at least one reinforcement panel comprises a nonwoven fabric, a multilayer laminate, a fluid-absorbing material, or a combination thereof. The at least one reinforcement panel can be backed by a fluid-repellent or fluid-impervious film layer. Further, the at least one reinforcement panel can be affixed to the base sheet via an adhesive, stitching, thermal bonding, or ultrasonic bonding.

The first hole containing portion is attached to a transparent portion of the drape.

In an additional embodiment, the first hole panel can include a nonwoven fabric, a multilayer laminate, a fluid-absorbing material, or a combination thereof.

In yet another embodiment, the portion of the drape covering the instrument array can have a thickness of from about 10 microns to about 60 microns.

In one more embodiment, the portion of the drape covering the instrument array can be a polyethylene film.

In a further embodiment, the hole in the first hole-containing portion of the first hole panel can have a generally circular shape. In one particular embodiment, the hole can be a femoral fenestration.

In another embodiment, the hole in the first hole-containing portion of the first hole panel can have a generally oval shape. In one particular embodiment, the hole can be a radial fenestration.

In one more embodiment, an adhesive can be positioned about a periphery of the drape to adhere the drape to the patient, the instrument array, or both. In one particular embodiment, the adhesive can be a pressure sensitive adhesive.

In another aspect of the present invention, there is provided a drape, as claimed in claim 13. Preferred embodiments of the invention as claimed in claim 13 are defined by dependent claims 14 and 15.

In one particular embodiment, the contrast delivery system has a touch screen display (CRT) that remains outside of the sterile field.

In an additional embodiment, the drape material is liquid impermeable.

Additional features and advantageous of the present invention will be revealed in the following detailed description. Both the foregoing summary and the following detailed description and examples are merely representative of the invention, and are intended to provide an overview for understanding the invention as claimed.

### BRIEF DESCRIPTION OF THE DRAWINGS

A full and enabling disclosure of the present invention to one skilled in the art, including the best mode thereof, is set forth more particularly in the remainder of the specification, including reference to the accompanying figures, in which:
Figure 1 is a view of a surgical drape covering a patient and an instrument array, showing the lines passing through the flap in the drape.
Figure 2 is a plan view of an embodiment of the drape showing the location of various features.
Figure 3 is a plan view of the holes and flap on the drape.
Figure 4 is a plan view of an alternative embodiment of the drape showing the location of various features.

### DETAILED DESCRIPTION

Reference now will be made in detail to various embodiments of the disclosure, one or more examples of which are set forth below. Each example is provided by way of explanation of the disclosure, not limitation of the disclosure. In fact, it will be apparent to those skilled in the art that various modifications and variations can be made in the present disclosure without departing from the scope of the disclosure. Thus, it is intended that the present disclosure cover such modifications and variations as come within the scope of the appended claims.

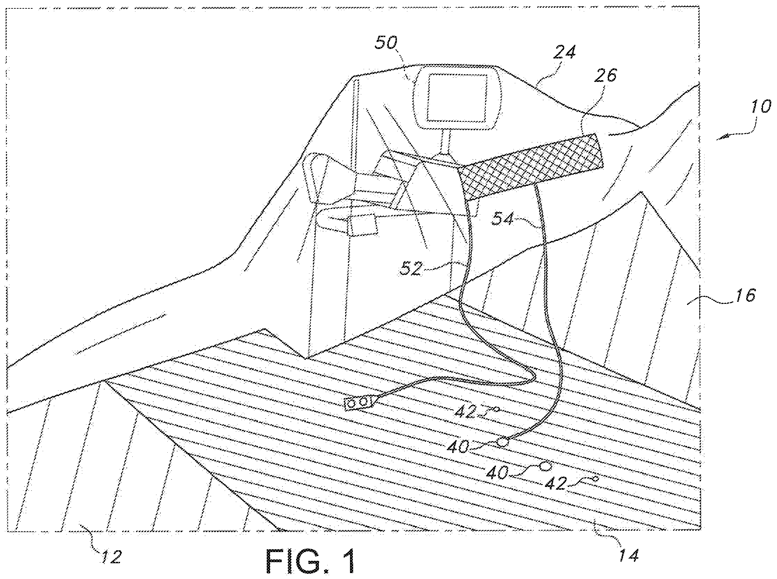

Surgical drapes formed in accordance with the present disclosure can generally possess any of a variety of sizes and shapes, depending on the particular use of the drape and on its desired properties. For example, certain surgical drape configurations are described in U.S. Patent No. 6,055,987 to Griesbach, et al. Various embodiments of surgical drapes incorporating aspects of the disclosure are depicted in the figures as drapes 10 for covering a patient (Figure 1) during a surgical procedure. The drapes 10 may be formed of any material or combination of materials defining a drape sheet material commonly used in the art for disposable surgical drapes, garments, covers, and so forth.

In general, the drape sheet may be made from a wide variety of materials, including, for example, woven, reusable fabrics and nonwoven disposable fabrics or webs. Nonwoven materials suitable for use with the present disclosure include, for example, multilayer laminates such as a spunbond/meltblown/spunbond ("SMS") material. An example of a suitable fabric is disclosed in U.S. Patent No. 4,041,203 to Brock, et al.

As used herein the term "nonwoven fabric or web" means a web having a structure of individual fibers or threads that are randomly interlaid, but not in an identifiable manner or pattern as in a knitted fabric. Nonwoven fabrics or webs have been formed from many processes such as for example, meltblowing processes, spunbonding processes, and bonded carded web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91).

As used herein the term "spunbond fibers" or "spunbonded fibers" refers to small diameter fibers which are formed by extruding molten thermoplastic material as filaments from a plurality of fine, usually circular capillaries of a spinneret with the diameter of the extruded filaments then being rapidly reduced, for example, as in U.S. Patent No. 4,340,563 to Appel, et al., and U.S. Patent No. 3,692,618 to Dorschner, et al., U.S. Patent No. 3,802,817 to Matsuki, et al., U.S. Patent Nos. 3,338,992 and 3,341 ,394 to Kinney, U.S. Patent No. 3,502,763 to Hartman, and U.S. Patent No. 3,542,615 to Dobo, et al. Spunbond fibers are generally not tacky when they are deposited onto a collecting surface. Spunbond fibers are generally continuous and have average diameters larger than 7 microns, more particularly, between about 10 and 20 microns.

As used herein the term "meltblown fibers" means fibers formed by extruding a molten thermoplastic material through a plurality of fine, usually circular, die capillaries as molten threads or filaments into converging high velocity, usually hot, gas (e.g., air) streams that attenuate the filaments of molten thermoplastic material to reduce their diameter, which may be to microfiber diameter.
Thereafter, the meltblown fibers are carried by the high velocity gas stream and are deposited on a collecting surface to form a web of randomly disbursed meltblown fibers. Such a process is disclosed, for example, in U.S. Patent No. 3,849,241 to Butin, et al. Meltblown fibers are microfibers that may be continuous or discontinuous, are generally smaller than 10 microns in average diameter, and are generally tacky when deposited onto a collecting surface.

As used herein "multilayer laminate" means a laminate wherein some of the layers are spunbond and some meltblown such as a spunbond/meltblown/spunbond (SMS) laminate and others as disclosed in U.S. Patent No. 4,041 ,203 to Brock, et al., U.S. Patent No. 5,169,706 to Collier, et al., U.S. Patent No. 5,145,727 to Potts, et al., U.S. Patent No. 5,178,931 to Perkins, et al., and U.S. Patent No. 5,188,885 to Timmons, et al. Such a laminate may be made by sequentially depositing onto a moving forming belt first a spunbond fabric layer, then a meltblown fabric layer and lastly another spunbond layer, followed by bonding the laminate in a manner described below. Alternatively, the fabric layers may be made individually, collected in rolls, and combined in a separate bonding step. Such fabrics usually have a basis weight of from about 0.1 osy to 12 osy (6 to 400 gsm), or more particularly from about 0.75 osy to about 3 osy (25 to 100 gsm). Multilayer laminates may also have various numbers of meltblown layers or multiple spunbond layers in many different configurations and may include other materials like films or coform materials (e.g., SMMS, SM, SFS, etc.).

As used herein, the term "coform" means a process in which at least one meltblown diehead is arranged near a chute through which other materials are added to the web while it is forming. Such other materials may be pulp, superabsorbent particles, cellulose or staple fibers, for example. Coform processes are shown in U.S. Patent No. 4,818,464 to Lau and U.S. Patent No. 4,100,324 to Anderson, et al. Webs produced by the coform process are generally referred to as coform materials.

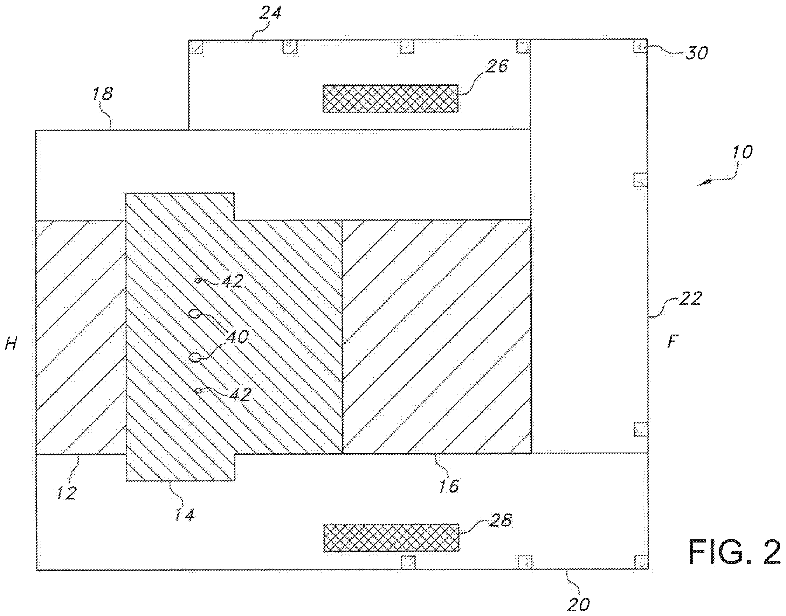

In one embodiment, the drape 10 includes femoral fenestration openings 40 and radial fenestrations 42 that can be placed over an operating site during surgery as is well known in the art. The fenestrations 40, 42 have a size, shape, and location that vary as a function of the particular type of surgical procedure for which the drape 10 is intended. For example, drapes intended for use in femoral angiography procedures may include one or two generally circular fenestrations 40, about 12 to about 13 mm in diameter, as indicated in Figure 2. Meanwhile, the radial fenestrations 42 are generally oval shaped and about 12 mm by about 7 mm to 8 about mm in diameter.

Instrument arrays may be used to monitor the progress of surgery or to administer fluids, like angiography contrast fluid, to a patient. The instrument array may contain, for example, a tough screen display (CRT) and other associated instrumentation to deliver the contrast fluid. A relatively new device is being used in angiography and involves the use of such an instrument array. This device, known as the ACIST CVi contrast delivery system, injects the contrast fluid via a high pressure line from the ACIST system and is controlled by a simple hand control piece, therefore making it less strenuous on the user to infuse. In addition, any remaining contrast media can be used for another patient as it is stored in a small glass bottle on the ACIST system, outside of the patient field. The ACIST system has a touch screen CRT and lines to deliver the fluid to the patient and a line to allow for the hand control of the fluid flow.

The drape 10 provided herein, which can be used in conjunction with the ACIST system, should include viewing panels (e.g., items 18, 20, 22, 24 in Figure 2) made of a transparent, fluid resistant material, such as polyethylene film, to cover the patient and the instrument array 50 and for easy viewing of the instrument array 50 through the transparent film as illustrated in Figure 1. Commonly used transparent film is generally from about 10 microns to about 60 microns in thickness, more particularly about 40 microns in thickness.

The surgical drape 10 of the present disclosure may include reinforcement panels 12, 14, 16 superimposed on and affixed in any suitable and appropriate manner to a base sheet, or bonded to the edges of the other panels (e.g., viewing panels 18, 20, 22, 24). The width and length and materials of construction of the reinforcement panels may vary depending on the intended use of the drape 10. The reinforcement panels may be formed from a variety of materials, such as nonwoven fabrics, multilayer laminates (e.g., SMS), fluid-absorbing materials and combinations thereof. The reinforcement panels may be backed by a fluid-repellent or fluid-impervious film layer. The film-layer side or lower surface of the reinforcement panels may be secured to the upper surface of a base sheet by any conventional means, including adhesive, stitching, thermal or ultrasonic bonding techniques. The upper surface of the reinforcement panels remains exposed and available to absorb fluids emitted from the surgical site. The fluid-impervious film layer prevents the passage of blood and other body fluids through the reinforcement panels and a base sheet, if present.

Strips or squares of adhesive 30 may be positioned around the periphery of the drape 10 to adhere the drape to the patient or to the instrument array 50. The tacky and pressure-sensitive adhesives used may be of any biologically acceptable adhesive. Examples of such adhesive materials are described in U.S. Patent No. 3,669,106 to Schrading, et al.

Some or all of the materials used to form the drape may be constructed so as to be hydrophilic or hydrophobic, and may be chemically treated to achieve the desired water absorbency properties. For instance, one or more materials may be treated with a surfactant in a manner such as described in U.S. Patent No, 5,540,979 to Yahiaoui, et al.

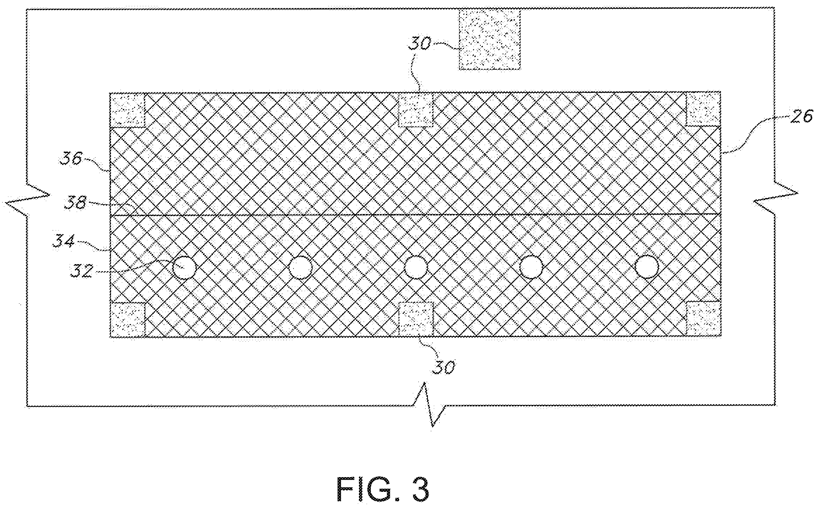

Referring to the figures, there is visible an instrument array 50 below a viewing panel 24 section of the drape 10 in Figure 1. The array 50 has two lines 52, 54 that are run through the pre-formed holes 32 in the first hole panel 26 of the drape 10. (Note that the first hole panel 26 and a small area of surrounding drape 10 are shown enlarged in Figure 3.) One of the lines is the controller line 52 and the other is the high pressure fluid line 54 that continues to the patient through one of the fenestrations 40. The first hole panel 26 has a flap 36 and a hole containing section 34. The flap 36 is only connected to the drape 10 at the fold 38. In use, the flap 36 is lifted and the lines 52, 54 inserted through any of the holes 32 and run to the array 50. The flap 36 folds down over the hole containing section 34 of the first hole panel 26 to shield the lines 52, 54 and the holes 32 and so maintain the sterile field of the patient after the lines have been inserted through the holes 32. The hole panel(s) may be made of the same materials as the reinforcement panel(s), as discussed above.

The first hole panel 26 is shown in Figure 2 as located on the upper side of the drape 10 since this is the most common location of the array 50 for surgery. Individual physician preference or other factors may dictate the array 50 be placed at another location. For this reason a second hole panel 28 is also provided on the other (opposite) side of the drape 10. Since the second hole panel 28 is on the opposite side of the patient, it is rotated 180 degrees so that the flap 36 folds down towards the patient. The two hole panels 26, 28 need not be the same size nor contain the same number of holes 32. In some embodiments the first hole panel 26 has five holes 32 and the second hole panel 28 has three holes 32, for example. The hole containing sections in this view are shown with the flaps closed so the holes are not visible.

In one embodiment (Figure 2), the drape has an overall length of about 340 cm in its longest dimension. At its head end (H in Figure 2) it is about 260 cm wide. The foot end (F in Figure 2) width is about 295 cm. The reinforcement panels 12, 14 and 16 have a combined length of about 275 cm with panel 14 being slightly wider by about 15 cm on each side as compared to reinforcement panels 12 and 16. The panels 12, 14, 16 may desirably be made of an SMS fabric with an absorbent layer added to panel 14 to aid in controlling blood and other fluids. The viewing panel 24 has a width of about 50 cm and ends about 150 cm short of the head end.

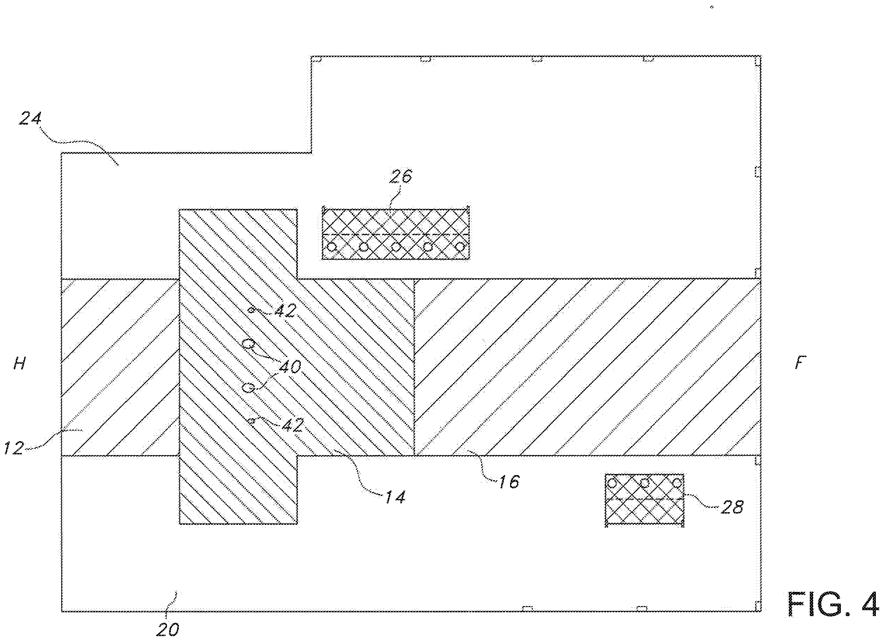

In another embodiment (Figure 4), the drape has an overall length of about 363 cm in its longest dimension. At its head end (H in Figure 4) it is about 238 cm wide. The foot end (F in Figure 4) width is about 288 cm. The reinforcement panels 12, 14 and 16 extend for the entire length of the drape (about 363 cm) so that there is no continuous transparent portion along the head or foot of the drape. Part of reinforcement panel 14 is slightly wider by about 35.5 cm on each side as compared to reinforcement panels 12 and 16, which are both about 91 cm wide. The widened area of panel 14 extends for about 61 cm along its length. The reinforcement panels 12, 14, 16 may desirably be made of an SMS fabric with an absorbent layer added to reinforcement panel 14 to aid in controlling blood and other fluids. There is a transparent viewing panel 24 that is about 363 cm long and that has a width of about 115 cm at the foot end and about 65 cm at the head end, reducing in width about 130 cm short of the head end. The alternative transparent viewing panel 20 is about 81 cm wide and about 363 cm long. The first hole panel 26 hole containing section 34 is about 76 cm long and about 12.5 cm wide and the flap 36 is the same size. The second hole panel 28 hole containing section is about 41 cm long and about 12.5 cm wide and the flap is the same size. The hole containing sections in this view are shown with the flaps open so the holes are visible.

The disclosed drape enables the user to pass lines from the sterile to non-sterile fields through the drape. It also allows a sterile user (i.e., a nurse) the option to set up the system without the assistance of a non-sterile nurse and allows the user to cover and use the touchscreen from the ACIST CVi system using only the patient drape and without the need for additional screen covers.

It should be appreciated by those skilled in the art that various modifications and variations can be made to the embodiments illustrated and described herein without departing from the scope of the invention, as set out in the appended claims. It is intended that the disclosure include such modifications and variations as come within the scope of the appended claims.

## Claims

1. A drape (10) for use during surgery on a patient, wherein the drape is configured for covering at least a portion of the patient during surgery and for covering an instrument array (50), wherein a portion of the drape (18, 20, 22, 24) covering the instrument array is transparent, and wherein the drape comprises a first hole panel (26) comprising a first hole containing portion (34) and a flap (36), wherein said first hole containing portion (34) is attached to a transparent portion (18, 20, 22, 24) of said drape, wherein a line (52, 54) for the delivery of a fluid to the patient can pass from the instrument array (50) through a hole (32) in the first hole containing portion (34) to the patient, and wherein the flap (36) is configured to cover the first hole containing portion (34).

2. The drape of claim 1, wherein said instrument array (50) is a contrast delivery system for angiography.

3. The drape of claim 1 or 2, wherein said drape includes a liquid impermeable material.

4. The drape of any of the foregoing claims, wherein said drape comprises a second hole panel (28) comprising a second hole containing portion in an alternative location of said drape from said first hole containing portion (34).

5. The drape of any of the foregoing claims, wherein said drape includes a base sheet and at least one reinforcement panel (21, 14, 16), wherein the at least one reinforcement panel comprises a nonwoven fabric, a multilayer laminate, a fluid-absorbing material, or a combination thereof, optionally wherein the at least one reinforcement panel is backed by a fluid-repellent or fluid-impervious film layer, optionally wherein the at least one reinforcement panel is affixed to the base sheet via an adhesive, stitching, thermal bonding, or ultrasonic bonding.

6. The drape of any of the foregoing claims, wherein said first hole panel (26) comprises a nonwoven fabric, a multilayer laminate, a fluid-absorbing material, or a combination thereof.

7. The drape of any of the foregoing claims, wherein the portion of the drape covering the instrument array (50) has a thickness of from about 10 microns to about 60 microns,

8. The drape of any of the foregoing claims wherein the portion of the drape covering the instrument array (50) is a polyethylene film.

9. The drape of any of the foregoing claims, wherein the hole (32) in the first hole-containing portion (34) of the first hole panel (26) has a generally circular shape, optionally wherein the hole is a femoral fenestration.

10. The drape of any of the foregoing claims, wherein the hole (32) in the first hole-containing portion (34) of the first hole panel (26) has a generally oval shape, optionally wherein the hole is a radial fenestration.

11. The drape of any of the foregoing claims, wherein an adhesive (30) is positioned about a periphery of the drape to adhere the drape to the patient, the instrument array, or both.

12. The drape of claim 11, wherein the adhesive (30) is a pressure sensitive adhesive.

13. A drape (10) for use during an angiography procedure, wherein the drape (10) is configured for covering at least a portion of a patient and for covering a contrast delivery system, wherein a portion of the drape (18, 20, 22, 24) covering the contrast delivery system is transparent, wherein the drape comprises a hole panel (26) comprising a hole containing portion (34) and a flap (36), wherein said first hole containing portion (34) is attached to a transparent portion (18, 20, 22, 24) of said drape, wherein a fluid delivery line (54) and a controller line (52) can pass from the contrast delivery system through holes (32) in the hole containing portion (34), and wherein the flap (36) can fold down over the fluid delivery line (54) and the controller line (52) where the fluid delivery line (54) and the controller line (53) pass through the hole containing portion (34).

14. The drape of claim 13, wherein said contrast delivery system has a touch screen display (CRT) that remains outside of the sterile field.

15. The drape of claim 13 or 14, wherein said drape material is liquid impermeable.

## Patentansprüche

1. Abdecktuch (10) zur Verwendung während einer Operation an einem Patienten, wobei das Abdecktuch zum Abdecken mindestens eines Abschnitts des Patienten während der Operation und zum Abdecken einer Instrumentenanordnung (50) konfiguriert ist, wobei ein Abschnitt des Abdecktuchs (18, 20, 22, 24), der die Instrumentenanordnung abdeckt, transparent ist, und wobei das Abdecktuch eine erste Lochbahn (26) umfasst, die einen ersten Lochaufnahmeabschnitt (34) und eine Klappe (36) umfasst, wobei der erste Lochaufnahmeabschnitt (34) an einem transparenten Abschnitt (18, 20, 22, 24) des Abdecktuchs befestigt ist, wobei eine Leitung (52, 54) zur Abgabe eines Fluids an den Patienten von der Instrumentenanordnung (50) durch ein Loch (32) in dem ersten Lochaufnahmeabschnitt (34) zum Patienten verlaufen kann, und wobei die Klappe (36) konfiguriert ist, den ersten Lochaufnahmeabschnitt (34) abzudecken.

2. Abdecktuch nach Anspruch 1, wobei die Instrumentenanordnung (50) ein Kontrastmittelabgabesystem für die Angiographie ist.

3. Abdecktuch nach Anspruch 1 oder 2, wobei das Abdecktuch ein flüssigkeitsundurchlässiges Material einschließt.

4. Abdecktuch nach einem der vorstehenden Ansprüche, wobei das Abdecktuch eine zweite Lochbahn (28) umfasst, die einen zweiten Lochaufnahmeabschnitt an einer alternativen Stelle des Abdecktuchs von dem ersten Lochaufnahmeabschnitt (34) umfasst.

5. Abdecktuch nach einem der vorstehenden Ansprüche, wobei das Abdecktuch eine Basislage und mindestens eine Verstärkungsbahn (21, 14, 16) umfasst, wobei die mindestens eine Verstärkungsbahn ein Vliesgewebe, ein mehrschichtiges Laminat und ein fluidabsorbierendes Material oder eine Kombination davon umfasst, wobei wahlweise die mindestens eine Verstärkungsbahn mit einer fluidabweisenden oder fluidundurchlässigen Filmschicht hinterlegt ist, wobei die mindestens eine Verstärkungsbahn wahlweise durch einen Klebstoff, eine Naht, thermische Verklebung oder Ultraschallverklebung an der Basislage befestigt ist.

6. Abdecktuch nach einem der vorstehenden Ansprüche, wobei die erste Lochbahn (26) ein Vliesgewebe, ein mehrschichtiges Laminat, ein fluidabsorbierendes Material oder eine Kombination davon umfasst.

7. Abdecktuch nach einem der vorstehenden Ansprüche, wobei der Abschnitt des Abdecktuchs, der die Instrumentenanordnung (50) abdeckt, eine Dicke von etwa 10 Mikrometer bis etwa 60 Mikrometer aufweist.

8. Abdecktuch nach einem der vorstehenden Ansprüche, wobei der Abschnitt des Abdecktuchs, der die Instrumentenanordnung (50) abdeckt, ein Polyethylenfilm ist.

9. Abdecktuch nach einem der vorstehenden Ansprüche, wobei das Loch (32) in dem ersten Lochaufnahmeabschnitt (34) der ersten Lochbahn (26) eine im Allgemeinen kreisförmige Form aufweist, wobei das Loch wahlweise eine femorale Fensterung ist.

10. Abdecktuch nach einem der vorstehenden Ansprüche, wobei das Loch (32) in dem ersten Lochaufnahmeabschnitt (34) der ersten Lochbahn (26) eine im Allgemeinen ovale Form aufweist, wobei das Loch wahlweise eine radiale Fensterung ist.

11. Abdecktuch nach einem der vorstehenden Ansprüche, wobei ein Klebstoff (30) um einen Umfang des Abdecktuchs positioniert ist, um das Abdecktuch an dem Patienten, der Instrumentenanordnung oder beiden zu befestigen.

12. Abdecktuch nach Anspruch 11, wobei der Klebstoff (30) ein Haftklebstoff ist.

13. Abdecktuch (10) zur Verwendung während eines Angiographieeingriffs, wobei das Abdecktuch (10) zum Abdecken mindestens eines Abschnitts eines Patienten und zum Abdecken eines Kontrastmittelabgabesystems konfiguriert ist, wobei ein Abschnitt des Abdecktuchs (18, 20, 22, 24), der das Kontrastmittelabgabesystem abdeckt, transparent ist, wobei das Abdecktuch eine Lochbahn (26) umfasst, die einen Lochaufnahmeabschnitt (34) und eine Klappe (36) umfasst, wobei der erste Lochaufnahmeabschnitt (34) an einem transparenten Abschnitt (18, 20, 22, 24) des Abdecktuchs befestigt ist, wobei eine Fluidabgabeleitung (54) und eine Steuerleitung (52) vom Kontrastmittelabgabesystem durch Löcher (32) in dem Lochaufnahmeabschnitt (34) verlaufen können und wobei die Klappe (36) über die Fluidabgabeleitung (54) und die Steuerleitung (52) nach unten klappen kann, wobei die Fluidabgabeleitung (54) und die Steuerleitung (53) durch den Lochaufnahmeabschnitt (34) verlaufen.

14. Abdecktuch nach Anspruch 13, wobei das Kontrastmittelabgabesystem eine Touchscreen-Anzeige (CRT) aufweist, die außerhalb des sterilen Feldes bleibt.

15. Abdecktuch nach Anspruch 13 oder 14, wobei das Abdecktuch ein flüssigkeitsundurchlässig ist.

## Revendications

1. Champ opératoire (10) destiné à être utilisé pendant une intervention chirurgicale sur un patient, dans lequel le champ opératoire est configuré pour recouvrir au moins une partie du patient pendant l'intervention chirurgicale et pour recouvrir un réseau d'instruments (50), dans lequel une partie du champ opératoire (18, 20, 22, 24) recouvrant le réseau d'instruments est transparent, et dans lequel le champ opératoire comprend un premier panneau d'orifices (26) comprenant une première partie contenant des orifices (34) et un rabat (36), dans lequel ladite première partie contenant des orifices (34) est fixée à une partie transparente (18, 20, 22, 24) dudit champ opératoire, dans lequel une voie (52, 54) destinée à l'administration d'un liquide au patient peut passer du réseau d'instruments (50) à travers un orifice (32) dans la première partie contenant des orifices (34) au patient, et dans lequel le rabat (36) est configuré pour recouvrir la première partie contenant des orifices (34).

2. Champ opératoire selon la revendication 1, dans lequel ledit réseau d'instruments (50) est un système d'administration de produit de contraste destiné à une angiographie.

3. Champ opératoire selon la revendication 1 ou 2, dans lequel ledit champ opératoire inclut un matériau imperméable aux liquides.

4. Champ opératoire selon l'une quelconque des revendications précédentes, dans lequel ledit champ opératoire comprend un second panneau d'orifices (28) comprenant une seconde partie contenant des orifices dans un autre endroit dudit champ opératoire de ladite première partie contenant des orifices (34).

5. Champ opératoire selon l'une quelconque des revendications précédentes, dans lequel ledit champ opératoire inclut une feuille de base et au moins un panneau de renforcement (21, 14, 16), dans lequel l'au moins un panneau de renfoncement comprend un tissu non tissé, un stratifié multicouche, un matériau absorbant les liquides ou une combinaison de ceux-ci, facultativement dans lequel l'au moins un panneau de renforcement est soutenu par une couche de film repoussant les liquides ou imperméable aux liquides, facultativement dans lequel l'au moins un panneau de renforcement est collé à la feuille de base par l'intermédiaire d'un adhésif, de sutures, de thermocollage ou de soudage par ultrasons.

6. Champ opératoire selon l'une quelconque des revendications précédentes, dans lequel ledit premier panneau d'orifices (26) comprend un tissu non tissé, un stratifié multicouche, un matériau absorbant les liquides ou une combinaison de ceux-ci.

7. Champ opératoire selon l'une quelconque des revendications précédentes, dans lequel la partie du champ opératoire recouvrant le réseau d'instruments (50) présente une épaisseur d'environ 10 microns à environ 60 microns.

8. Champ opératoire selon l'une quelconque des revendications précédentes dans lequel la partie de champ opératoire recouvrant le réseau d'instruments (50) est un film de polyéthylène.

9. Champ opératoire selon l'une quelconque des revendications précédentes, dans lequel l'orifice (32) dans la première partie contenant des orifices (34) du premier panneau d'orifices (26) présente une forme généralement circulaire, facultativement dans lequel l'orifice est une fenêtre fémorale.

10. Champ opératoire selon l'une quelconque des revendications précédentes, dans lequel l'orifice (32) dans la première partie contenant des orifices (34) du premier panneau d'orifices (26) présente une forme généralement ovale, facultativement dans lequel l'orifice est une fenêtre radiale.

11. Champ opératoire selon l'une quelconque des revendications précédentes, dans lequel un adhésif (30) est positionné autour d'une périphérie du champ opératoire pour faire adhérer le champ opératoire au patient, au réseau d'instruments ou au deux.

12. Champ opératoire selon la revendication 11, dans lequel l'adhésif (30) est un adhésif autocollant.

13. Champ opératoire (10) destiné à être utilisé pendant une procédure d'angiographie, dans lequel le champ opératoire (10) est configuré pour recouvrir au moins une partie d'un patient et pour recouvrir un système d'administration de produit de contraste, dans lequel une partie du champ opératoire (18, 20, 22, 24) recouvrant le système d'administration de produit de contraste est transparent, dans lequel le champ opératoire comprend un panneau d'orifice (26) comprenant une partie contenant des orifices (34) et un rabat (36), dans lequel ladite première partie contenant des orifices (34) est fixée à une partie transparente (18, 20, 22, 24) dudit champ opératoire, dans lequel une voie d'administration de liquide (54) et une voie de dispositif de régulation (52) peuvent passer du système d'administration de produit de contraste à travers des orifices (32) dans la partie contenant des orifices (34), et dans lequel le rabat (36) peut se replier sur la voie d'administration de liquide (54) et la voie du dispositif de régulation (52) où la voie d'administration de liquide (54) et la voie du dispositif de régulation (53) traversent la partie contenant des orifices (34).

14. Champ opératoire selon la revendication 13, dans lequel le système d'administration de produit de contraste est muni d'un écran d'affichage tactile (CRT) qui reste en dehors du champ stérile.

15. Champ opératoire selon la revendication 13 ou 14, dans lequel ledit matériel de champ opératoire est imperméable aux liquides.
